# EUROPEAN PATENT SPECIFICATION

(11) **EP 2 063 728 B1**
(45) Date of publication and mention of the grant of the patent: **15.09.2010**
(21) Application number: 07734373.9
(22) Date of filing: 24.04.2007
(51) Int. Cl.: A42B 3/32

(54) **CRASH HELMET WITH A FLUSH RECESSED VISOR HAVING A TILTING FRONT CHIN-VISOR PART THEREOF**
STURZHELM MIT EINEM BÜNDIGEN AUSGESPARTEN VISIER MIT EINEM VORDEREN KIPP-KINNVISIERTEIL
CASQUE DE PROTECTION POURVU D'UNE VISIERE RENFONCEE AFFLEURANTE COMPRENANT UNE PARTIE AVANT DE MENTONNIERE-VISIERE INCLINABLE

(30) Priority: 24.04.2006 IT MI20060820
(43) Date of publication of application: 03.06.2009
(73) Proprietor: Nava, Pier Luigi, 20040 Cornate d'Adda MI (IT)
(72) Inventor: Nava, Pier Luigi, 20040 Cornate d'Adda MI (IT)
(74) Representative: Cicogna, Franco
(86) International application number: PCT/IB2007/001055
(87) International publication number: WO 2007/122496

(56) References cited:
- EP-A1- 0 685 177
- EP-B1- 1 265 506
- DE-A1- 10 234 330
- DE-A1-102004 048 839
- US-A1- 2003 182 716

## Description

### BACKGROUND OF THE INVENTION

The present invention relates to a crash helmet having a flush recessed visor and a tilting chin rest-visor front portion.

The invention as herein disclosed comprises specifically designed operating mechanisms, to provide a flush type of helmet cap.

As is known, several crash helmets having an openable visor-chin rest front portion are already available, see for example EP 0 685 177 A1.

However, prior technical approaches have been found to be unsatisfactory from both the aesthetic and functional standpoint.

A first disadvantage of the prior crash helmets is that they comprise very complex mechanisms for driving the crash helmet operating movements.

All the above driving mechanisms are arranged on a side of the user ear region and comprise pivot pins, or lever systems, of a comparatively large size, which greatly project from the crash helmet cap pattern, with consequent unpleasant aesthetic results.

Moreover, they generate a lot of noises during the running, in particular just at the user ear region, thereby fatiguing and disturbing the user.

### SUMMARY OF THE INVENTION

Accordingly, the aim of the present invention is to provide a crash helmet having a tilting visor and chin rest assembly, which is substantially flush with the crash helmet outer cap, thereby providing great aesthetic and aerodynamic advantages.

Within the scope of the above mentioned aim, a main object of the invention is to provide such a crash helmet which is very reliable and safe in a crash or impact event and/or in a user failing accident.

The above mentioned aim and objects, as well as yet other objects, which will become more apparent hereinafter, are achieved by a crash helmet having a flush recessed visor, i.e. a visor flush with crash helmet cap and having a tilting front chin rest-visor portion, according to the enclosed claim 1.

Further important characteristics of the present invention are defined in the dependent claims.

Thus, the crash helmet according to the present invention provides a protective helmet in which the crash helmet visor and chin rest are adapted to be tilted, to be brought essentially flush with the crash helmet outer cap contour.

Accordingly, the crash helmet cap does not comprise projections, and, accordingly, has a continuous aerodynamic contour of very good aesthetic properties, better than that of the prior art crash helmets.

Moreover, the subject crash helmet is very safe in operation as it will become more apparent from the following disclosure of the invention.

A further advantage of the invention is that since the crash helmet does not comprise projections, in a falling accident, the crash helmet can slide on the ground, in a normal manner, without any projections which, if they would be impacted, would generate rotational acceleration effects, negatively affecting the user head and consequently the user brain, with great damages of the brain mass, very serious and much more dangerous than an impact perpendicular to the user skull.

### BRIEF DESCRIPTION OF THE DRAWINGS

The invention will be disclosed in a more detailed manner hereinafter, with reference to the accompanying drawings showing some preferred embodiments of the invention, and where:
Figure 1 shows a crash helmet according to the invention, in its main aspect, and as seen in a side elevation view;
Figure 2 shows a spreading, rotary and locking system associated with the subject crash helmet, with the movable chin rest being shows in a raised position, and including locking means for locking a side movable element, for disengaging the user ears;

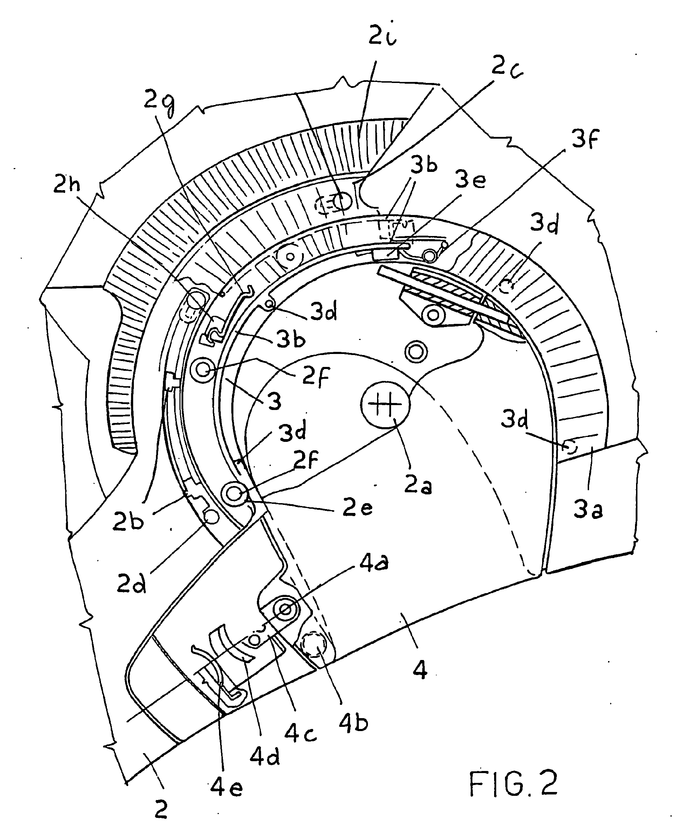

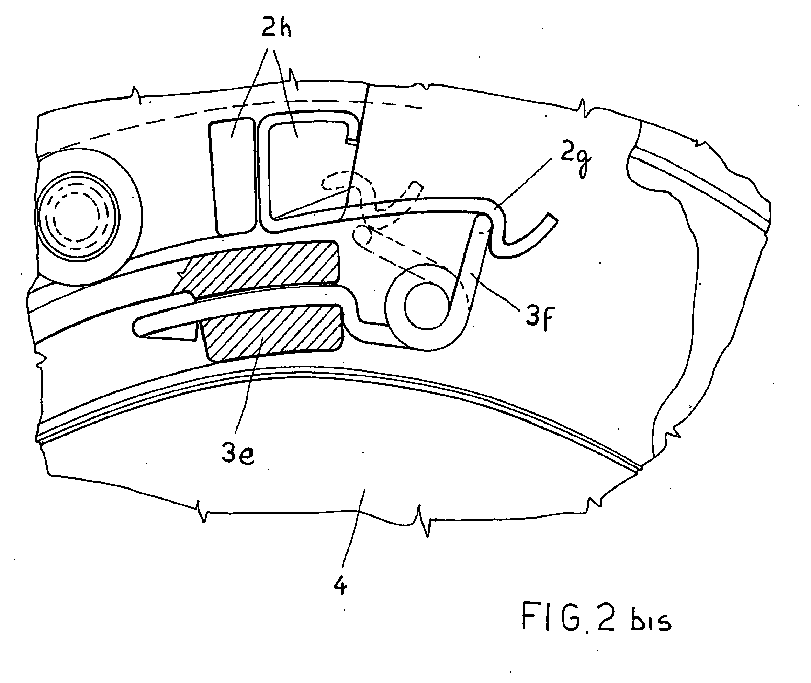

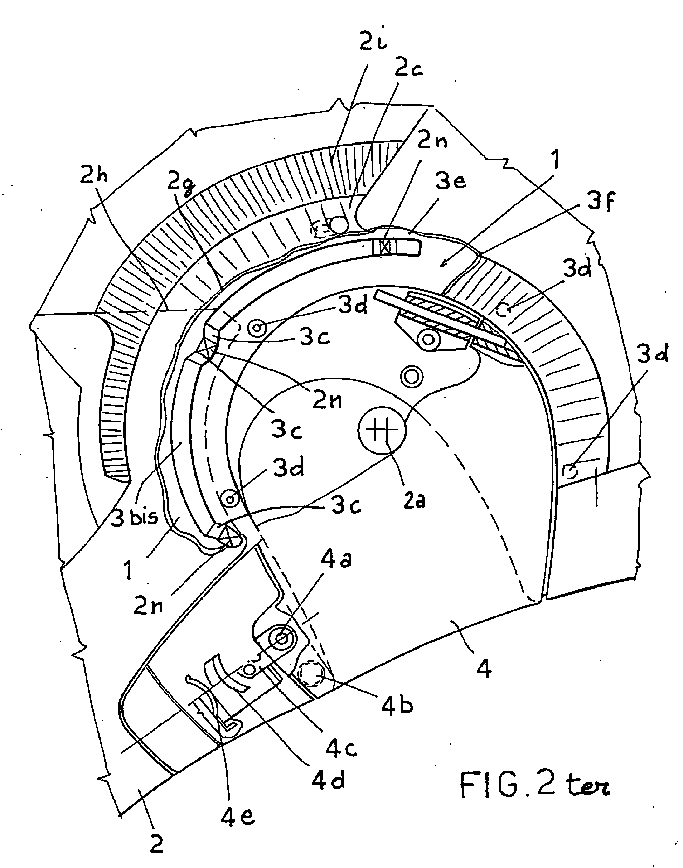

Figure 2 bis shows a detail of the resilient locking spring system included in the chin rest assembly, in a raised condition thereof;
Figure 2 ter shows, in a cross-sectioned side view, the crash helmet according to the present invention, including a sliding path for the crash helmet chin rest;

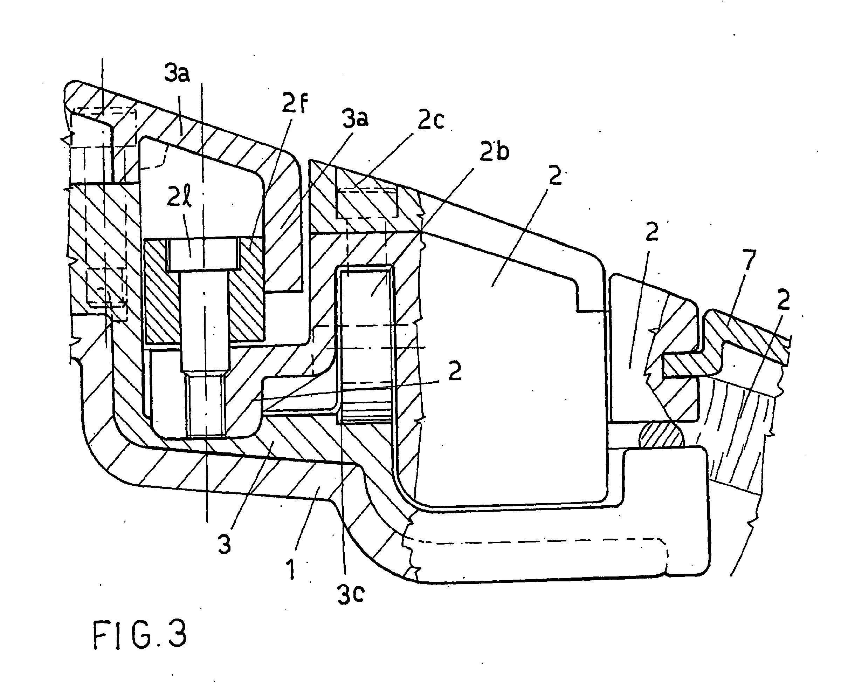

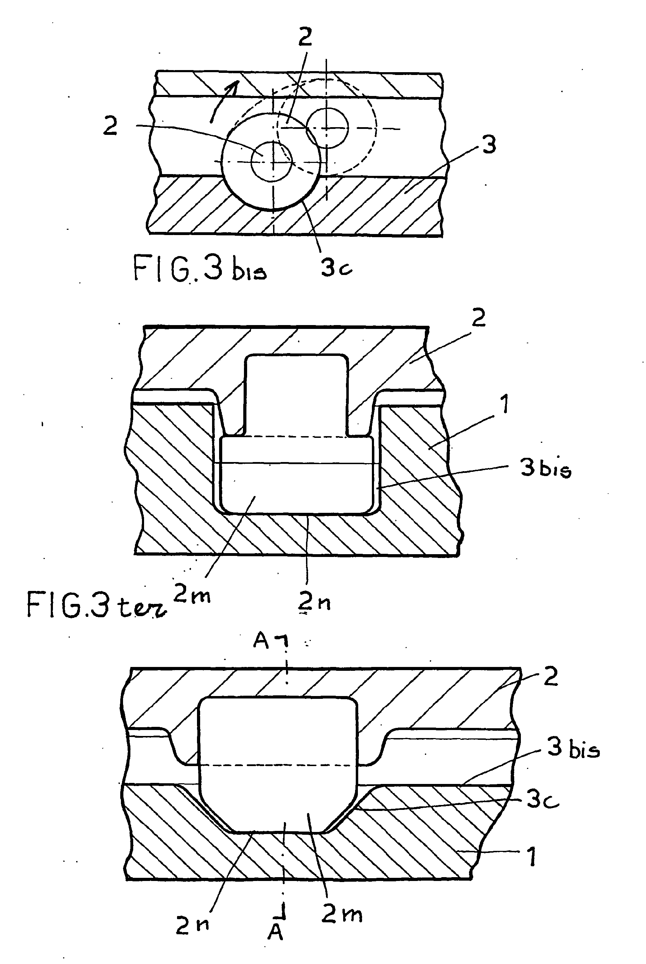

Figure 3 is a cross sectional view showing the full system of the crash helmet cap, base, rotary gears, visor holder gears and related visor;
Figure 3 bis shows a detail of the mechanism for spreading the side elements of the crash helmet chin rest portion, as the raising-rotary movement is started, and allowing the chin-rest portion to exit its flush recess or seat and turn backward;
Figure 3 ter is a cross sectional view showing the channel at a rest point of the skids 2n;

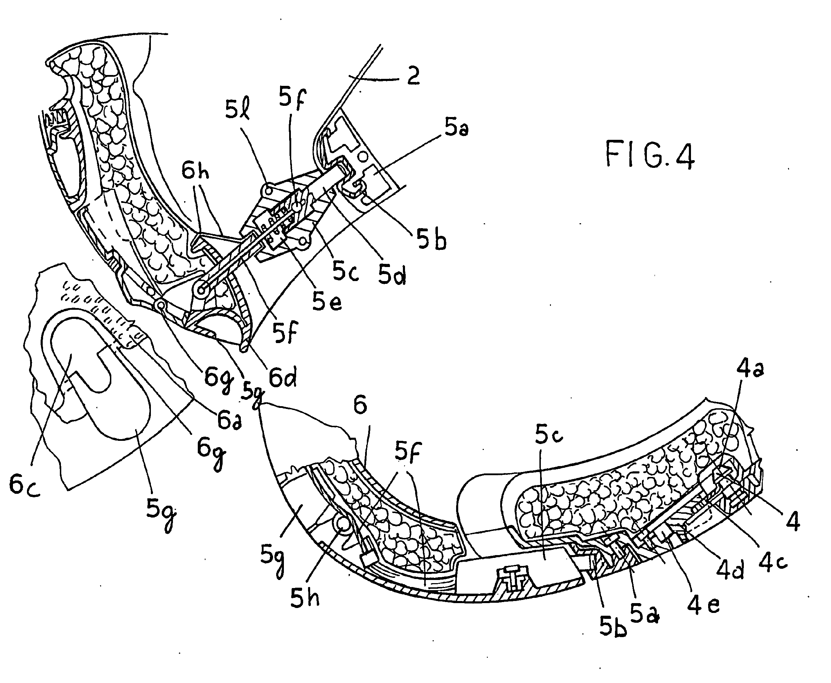

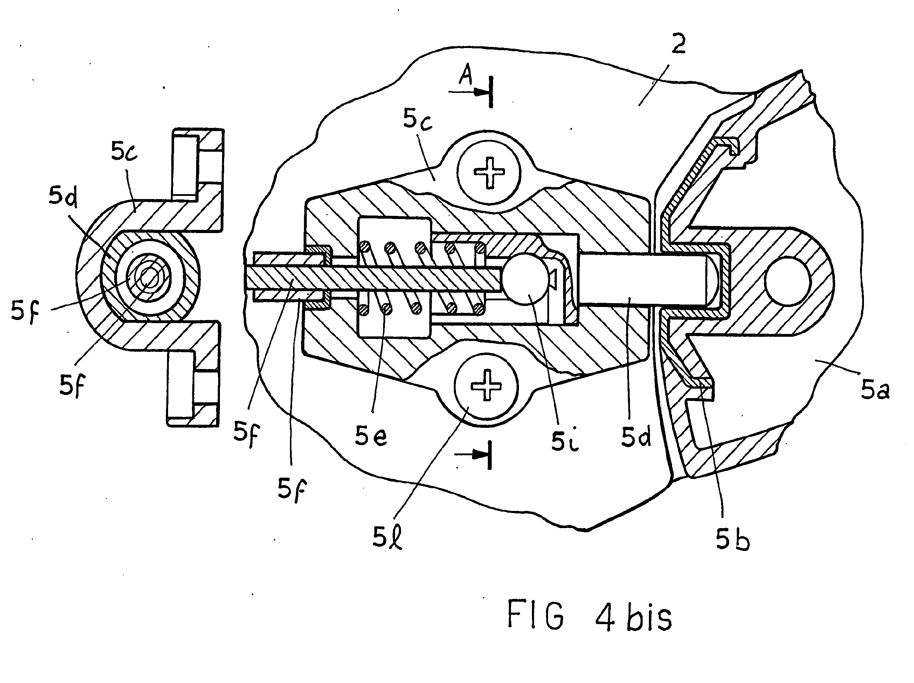

Figure 4 is a further cross sectional view showing the anchoring and locking system for anchoring and locking a movable ear element, the driving and opening system for the latter, the overall drive system of the locking elements for locking the chin-rest portion at a lowered position thereof, and the front driving elements for opening said locking element and driving a gate member for a front air blowing in, in addition to a flexible and upsettable spoiler element arranged inside the chin-rest portion;
Figure 4 bis shows a detail of the small block including the locking member or element and a cross-sectional view according to the cross-sectional plane A-A';

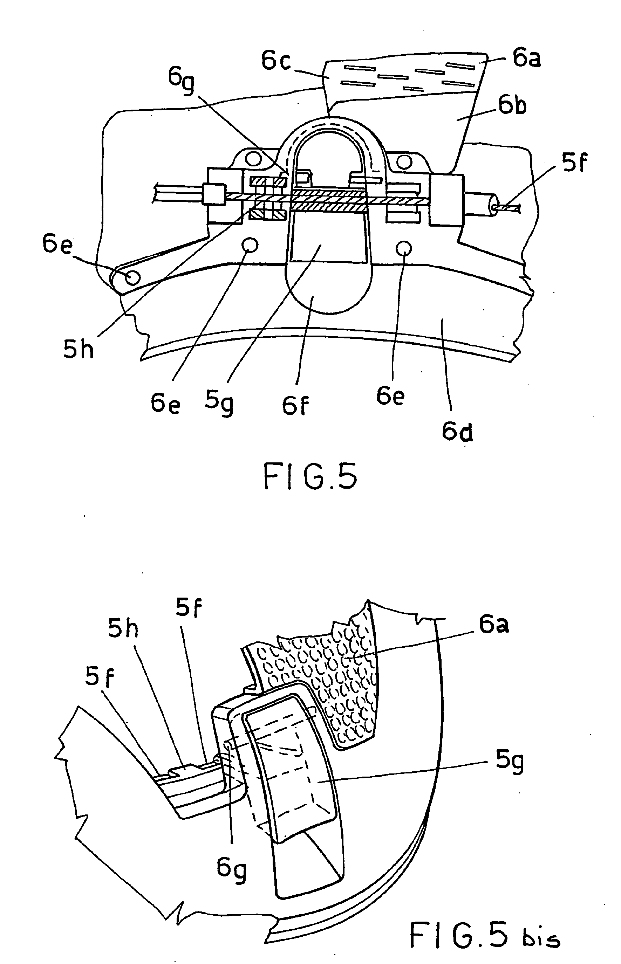

Figure 5 shows a front inner portion of the crash helmet with an assembled block for driving the locking elements, gate element, and thereby the flexible chin under portion is affixed to the crash helmet cap;
   and
Figure 5 bis is an outer view showing the front portion of the inventive crash helmet.

### DESCRIPTION OF THE PREFERRED EMBODIMENTS

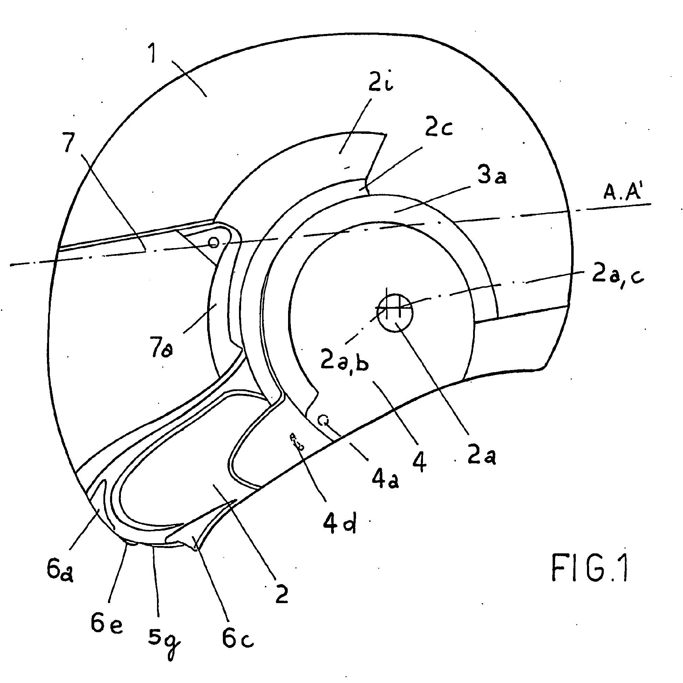

With reference to figure 1, a crash helmet is herein shown according to the invention, and as seen in a side elevation view.

More specifically, this figure also shows a tilting chin rest-visor assembly, including a movable chin rest-visor element 2, arranged flush with the crash helmet cap and having, at the end portions thereof, substantially circular sector shape elements 3, and a cover 3a, which elements are firmly anchored to the crash helmet cap 1.

Said crash helmet cap 1 is, in turn, so contoured as to receive therein restraining, spreading, sliding and locking elements, moving along substantially circular path patterns, and the ideal centers of rotation of which (generally indicated in figure 1 by the reference numbers 2a, 2ab and 2ac) are arranged inside a covering region which covers the user ear and at the bottom of the line A-A' shown in figure 1.

In the crash helmet according to the present invention, the sliding track for the visor support element has the same virtual rotary center 2ab as that of said chin rest portion, whereas the rotary center 2ac of the visor 7 is arranged at a position different from that of the virtual rotary center 2ab of the visor-chin rest assembly.

The crash helmet according to the present invention comprises moreover a crash helmet sector 3, made as a single piece with the chin rest assembly and including a visor holding sector 7a, covered by a flexible plate element 2i, allowing the flush visor 7 to be backward tilted and caused to slide, which element is arranged just at an outer circumference sector, on the outside of the rotary recess of the chin rest assembly primary element.

Thus, by using the above mentioned circular sector 3 it is possible to free the ear region from any mechanisms, thereby providing a larger space for the ear pavilions, and allowing an optional application of side movable elements 4.

Actually, side movable elements 4 are herein provided, optionally covering the ear region and which are independent from the rotary and guide system of the visor-chin rest movable element.

More specifically, said movable elements 4 (one for each portion) covering the user ears, are affixed to the crash helmet cap 1 by a rotary-opening and closing locking system, substantially arranged inside the system for tilting the visor-chin rest assembly, without interfering against the latter and with a substantially flush relationship with the crash helmet cap, thereby improving the crash helmet aesthetic aspect and operating characteristics.

In particular, said movable element 4 locking mechanism comprises, on each side, a metal hook element 4a mounted on the movable element 4 and which, during its closing element, will engage and backward drive a metal blade element 4c mounted on the crash helmet cap 1, and in a movable slider 4d including a pushing and return spring 4e.

Thus, with the blade elements 4c backward displaced, the hook element 4a will pass through and, under the urging force of the spring 4e, it will engage in the cut-out of the hook element, thereby providing the desired latching relationship; an antivibration plug 4b being herein moreover provided.

In the inventive crash helmet, the upward directed force provided by the chin rest portion, and the consequent backward rotary movement will provide, simultaneously, a spreading of the side end portions of the chin rest portion, thereby the latter will exit its recess or seat flush with the crash helmet cap, together with the visor assembly and rigidly therewith, thereby allowing the backward rotary movement.

In particular, the circular sector 3 comprises a plurality of spreading wheel elements, on each side, 2b, having respective pivot pins 2e and which, in a closure position, are housed in a lowermost recess 3c of the circular sector 3, as is clearly shown in figure 3bis, and which, by moving and exiting their recess will spread apart, simultaneously with rotary movement, the side portions of the chin rest assembly, thereby the latter will exit its recess or seat flush with the crash helmet cap, and holding said side portions spread apart even at the end of the upward movement.

From the figures it is further possible to show a cover 2c covering the spreading wheel elements 2b, as well as bayonet attachments 2d for coupling the cover 2c.

More specifically, the circular sector 3 comprises two small sliding rollers, on each side, as indicated by 2f, having respective pivot pins 21, said rollers 2f being restrained at a set position by a rib arrangement of the base 3b and a further rib 3a formed in said cover 2c, which ribs will provide a substantially circular guide coaxial with that of the spreading apart wheels 2b, and therealong they slide, to in turn rotate, about its ideal center 2ab, or 2ac, upward and backwards, the above mentioned chin rest-visor assembly, up to an end of stroke position thereof.

Said chin rest portion 2 comprises moreover, at at least a circular sector of an end portion thereof affixed in a restraining block 2h, a strip spring 2g, which is suitably contoured to overcome and latch, at an end stroke of its upward and backward displacements, a further round wire spring 3f arranged on the base of said circular sector 3.

Thus, the overall chin rest and visor assembly is held in a raised position thereof, in a very firm and resilient manner, thereby absorbing all the movement dynamic stresses.

As the user deliberately lowers the chin-rest assembly 2, the round wire spring 3f, during its downward movement, is driven along a circle sector, thereby being actually lowered, and consequently disengaged, after having followed a given pathlength, from the hook element of the strip spring 2g, and consequently making the chin rest-visor assembly free, to allow the downward movement to be continued up to a closure condition.

The chin rest-visor assembly comprises, at the two involved side regions thereof, a latching or locking system, of a firm and operatively reliable type, holding the assembly in its closing position.

The latching or locking system 5, shown in figure 4 bis, essentially comprises a male locking mechanism 5d and a female locking mechanism 5b, the latching element 5d being urged by a return spring 5e holding said latching element 5d and causing it to be always returned to a fully projecting position, and it also comprises a further pulling wire locking or latching element 5i, or Bowden operating cable 5f, in turn driven by a control lever 5g, engaged on a small block 5a including two clamping screws 51, at the chin rest front portion, coupled to the latching element by a Bowden's wire or cable, or the like, and simultaneously controlling the two latching elements, on the right and on the left, opening movement.

More specifically, during the assembly closing operation, the head portion of the latching element engages with a slanted chute portion, including a suitably contoured metal band 5b, mounted in the block 5a on the crash helmet cap, and operating as a restraining female portion.

During the closing movement, as the latching element head abuts on the inclined surface, it caused the latching element to be backward driven, while overcoming the urging of the spring 5e, while allowing, as the latching element is returned to its fully projecting position, said latching element to be engaged in the female part 5b thereby locking the chin rest assembly at a closing position thereof.

In particular, the Bowden cable 5f is held in a tensioned condition by two tension rollers 5h, as is clearly shown in figure 4, arranged at any desired suitable positions.

The latching element right and left systems, the latching element front control assembly, comprising the tension members and air inlet system gate, are all included in three blocks, coupled by said Bowden cable 5f and of which a block 6 is clamped at the front portion whereas the other right and left blocks 5 are clamped on the two sides at any desired suitable position.

As shown, the gate element 6b has the same rotary center 6g as that of the latching element control lever 5g.

The chin rest portion 6d is made of a flexible material, and said chin rest assembly also provides the corresponding portion for the recess or seat necessary to properly operate the control lever 5g which, at a lowered position thereof is of a sufficient duration to operate as an aerodynamic spoiler to prevent air from entering at the bottom, and which, as inward upturned, engages in a suitable recess, to be held flush with the inner contour of the chin rest assembly.

More specifically, figure 5 shows the front inner portion on which the latching element 6c and gate element 6b block is assembled, and thereby the flexible chin rest assembly 6d is also clamped to the crash helmet cap.

In this figure it is also possible to see the block clamping points 6e, together with a recess 6f for engaging the latching element control or driving lever 5g.

Figure 5 bis shows an outer view of the front part of the inventive crash helmet with its front air inlet 6a, the Bowden cable 5f control lever 5g and the tension rollers 5h.

All the above disclosed systems operate in a very simple and self-apparent manner, thereby providing a safe and easy operation.

More specifically, the user, to wear the crash helmet, as the latter is in a closed condition, must pull on the control lever 5g, to make the latching elements free or disengaged, and backward tilt the chin rest-visor assembly, which will be laterally moved away, thereby exiting its recess and, by a continuous type of movement, it will further backward turn to a stop condition, which is signaled by a click snap noise, due to the engagement or latching of the strip spring with the wire spring.

If the crash helmet further comprises the disclosed side elements, then the user may open the related sliders, by directly operating them thereby making free the space for allowing the user ear pavilions to pass therethrough, without any efforts or friction.

After having worn the crash helmet and closed the restraining belt, the user will further reclose, by a very simple operation, the side elements to cause them to be clamped to the crash helmet cap, which would be signaled by another click snap noise, just indicating to the user the obtained closing condition.

Thus, the user may run on his/her motorcycle with the chin rest assembly in a raised condition, without any problems, since air will be caused to pass between the chin rest assembly and top portion of the crash helmet cap, without generating any sailing effects.

If the user desires to close the chin rest assembly, then he/she will perform a very simple gripping operation by using a hand to lower the chin rest assembly thereby automatically unlatching or disengaging the spring restraining system.

Making now reference to figures 2 ter and 3 ter, they show a modified embodiment of the device shown in figures 2 bis.

Nor specifically, said figures 2 ter and 3 ter show a sliding track 3 bis, formed on the base portion or directly on the crash helmet cap 1.

This sliding track 3 bis, comprises different sliding surfaces, and is of a substantially circular configuration, and includes corresponding cam elements 3c.

Said cam elements will allow the chin rest assembly 2 to perform, during the opening operation, a composite continuous movement with a plurality of outward, frontward and upward lateral displacements.

The reference numbers 2n show the corresponding seats, respectively three for each side, of the siding skids 2m, with the latter being in a rest condition in their opening and closing positions.

In this connection it should be apparent that it would be possible to properly locate the chin rest assembly at different middle inclined positions.

At the end of stroke position, a further click snap noise will signal the user that the latching elements have been brought to a closing condition, and that the chin rest assembly constitutes now a firm and safe portion of the crash helmet.

It should be apparent that the invention must not be intended as limited only to motor cycle sport crash helmets: the invention, on the contrary, could also be advantageously used in each type of helmets.

In particular, for helmets not requiring a high impact absorption level, for example ski helmets, climbing helmets, horse-riding helmets, skating helmets, gliding helmets and other sport helmets.

Accordingly, the invention would be susceptible to several modifications and variations all entering within the inventive idea, as defined in the accompanying claims, the technical details being in turn susceptible to be changed depending on requirements.

Moreover, all the dimensions and parameter values and physical amounts as therein above mentioned, and the related used materials, must be intended only by way of a non limitative example of the inventive features.

### Reference number list

1. Crash helmet cap
2. Tilting chin rest assembly
   2a, 2ab, 2ac Ideal rotary centers
   2b Spreading apart wheel elements
   2c Spreading apart wheel element cover
   2d Bayonet attachments for clamping the cover
   2e Spreading apart wheel element pivot pin
   2f Circular sector sliding rollers
   2g Resilient locking strip spring
   2h Resilient strip spring restraining block
   2i Flexible plate
   21 Sliding wheel pivot pins
3. Circular sector element for housing the movable sector including the related guides and fittings
   3a Cover and corresponding guide rib
   3b Rib
   3c Cams
   3d Threaded or bayonet recesses for clamping the cover
   3e Small block
   3f Round wire spring
4. Movable side covering element for covering the user ears and corresponding closing system
   4a Hook latching element
   4b Anti-vibrating plug
   4c Metal blade
   4d Movable slider
   4e Flat spring element for restraining the slider at a set position
5. Latching element locking system with a single front control assembly
   5a Block with the metal strip female portion
   5b Metal band providing a recess for restraining the latching element or female latching element
   5c Body of the block bearing the latching element DX and SX mechanism
   5d Male latching element
   5e Latching element return spring
   5f Bowden cable
   5g Bowden cable control lever
   5h Tension rollers
   5i Latching element locking element
   51 Block clamping screws
6. Central block including a single control for the latching elements, gate element and related control assembly, and clamping the flexible chin rest assembly to the crash helmet
   6a Front air inlet grid
   6b Gate element
   6c Gate element opening and closing control assembly
   6d Flexible and reversing under-chin portion 6e Clamping points of the block
   6f Recess for engaging the latching element control lever
   6g Control lever and gate rotary pivot pin
   6h Flexible under-chin portion
7. Flush visor
   7a Flush visor holder sector

## Claims

1. A crash helmet of a type having a crash helmet cap (1) and a movable chin rest-visor assembly (2) including a tilting chin rest-visor front portion, said crash helmet cap (1) supporting restraining, spreading apart, sliding and locking elements for said assembly, said assembly further comprising, at the two end portions thereof, substantially circular sector shaped elements (3) firmly anchored to said crash helmet cap (1), **characterized in that** said movable chin rest-visor assembly (2) is arranged in a flush relationship with said crash element cap (1), that said restraining, spreading apart, sliding and locking elements are movable along substantially circular path patterns, having ideal rotary centers (2a, 2ab, 2ac) arranged inside a region suitable to cover a user ear, and that said circular sector shaped elements (3) allow to disengage the user ear regions of said crash element from a locking mechanism therefor, thereby providing an enhanced space for the ear pavilions and for an application of movable elements (4).

2. A crash helmet according to claim 1, **characterized in that** said movable elements (4) covering the user ear region are independent from the chin rest-visor movable element rotary and guide system.

3. A crash helmet according to claim 1, **characterized in that** said movable elements (4) covering said ears are clamped to the crash helmet cap by rotary opening means and locking means for providing a locking at a closing position, substantially inside the system allowing the visor-chin rest assembly to be tilted, without interfering against said visor-chin rest assembly, and substantially with a flush relationship with respect to the crash helmet cap contour.

4. A crash helmet according to claim 1, **characterized in that** said locking mechanism comprises a metal hook element (4a) assembled on said movable element (4) which, during its closing movement, engages and backward drives a blade element (4c) mounted on said crash element cap (1), said locking mechanism also comprising a movable slider (4d) including an urging and return spring (4e), to cause said blade element (4c) to be backward driven thereby allowing said hook element (4a) to pass through, and, being urged by said spring (4e), to enter a cut-out of said hook element (4a) thereby providing a locking relationship.

5. A crash helmet according to the preceding claims, **characterized in that** the upward urging of said chin rest assembly, and the corresponding backward rotary movement, simultaneously provide a spreading apart of the side end portions of said chin rest assembly, said chin rest assembly being disengaged from its recess, flush with the crash helmet, together with the visor assembly integral therewith, thereby allowing a backward rotary movement to be performed.

6. A crash helmet according to claim 1, **characterized in that** said circular sector elements (3) comprise wheel elements (2b) on each side thereof which wheel elements, at a closed condition thereof, are engaged in a bottom recess (3c) of said circular sector elements (3) and which, as they are driven and exit the recess therefor, simultaneously rotatively spread apart the side portions of the chin rest assembly thereby causing the latter to be disengaged from the crash helmet cap flush recess therefor, and holding said side portions in a spread apart condition even at an upward end of stroke position.

7. A crash helmet according to claim 1, **characterized in that** said circular sector elements (3) comprise two wheel elements on each side thereof (2f), said wheel elements being restrained by a restraining ribs forming a substantially circular guiding pattern, coaxial to that of the spreading apart wheel elements (2b), and therealong they slide thereby causing said chin rest-visor assembly to be turned about its ideal rotary center (2ab or 2ac) upward and backward, to achieve an end of stroke or limit position thereof.

8. A crash helmet according to claim 1, **characterized in that** said chin rest assembly (2) comprises a first strip spring (2g) cooperating with a second round wire spring (3f) to firmly and resiliently hold said chin rest-visor assembly at a raised position thereof.

9. A crash helmet according to claim 1, **characterized in that** said crash helmet comprises a sliding track for the visor support assembly, said sliding track having a same virtual rotary center as that of said chin rest assembly (2ab).

10. A crash helmet according to claim 9, **characterized in that** the visor rotary center is arranged at a different position from that of the visor-chin rest assembly virtual rotary center (2ac).

11. A crash helmet according to claim 1, **characterized in that** said circular sector element (3) forming a single body with said chin rest assembly, is so designed as to allow said flush visor to be backward tilted and slidably moved.

12. A crash helmet according to claim 1, **characterized in that** said chin rest-visor assembly comprises, on corresponding side regions thereof, firm and safe locking means designed for holding in a closed position said assembly, said locking means substantially comprising a male latching element (5d) and a female latching element (5b), said male latching element (5d) being urged by an urging spring (5e) holding and always recovering said male latching element to a fully projecting position, and a further latching or locking element (5i) and a Bowden cable (5f), controlled by a controlling lever (5g) arranged on a supporting block at the front portion of the chin rest assembly.

13. A crash helmet according to claim 12, **characterized in that** said male latching element (5d) comprises a head portion engaging with an inclined chute pattern, comprising metal band (5b) supported in said block (5a) on the crash helmet cap, and operating as a restraining female latching element.

14. A crash helmet according to claim 12, **characterized in that** said cable (5f) is held in a tensioned condition by two tension rollers (5h).

15. A crash helmet according to claims 12, **characterized in that** the right and left systems of said latching elements, the front controlling arrangement of said latching elements, including said tension elements and the gate element of the air inlet system, are all included in three blocks, coupled by said Bowden cable (5f).

16. A crash helmet according to claims 12, **characterized in that** said crash helmet comprises a gate element (6b) having the same rotary center (6g) as that of said control lever (5g).

17. A crash helmet according to claim 12, **characterized in that** a portion of an under chin assembly (6d), is made of a flexible material, and also constitutes a part including the recess for allowing said control lever (5g) to operate and which, at a lowered position, has a sufficient length to operate as an aerodynamic spoiler for preventing bottom air from entering and which, as it is inward turned, engages in an engagement seat, thereby being held in a flush relationship with an inner contour of the chin rest assembly.

18. A crash helmet according to claim 1, **characterized in that** said crash helmet further comprises a sliding track (3 bis), comprising cam elements (3c) allowing said chin rest assembly (2) to provide a composite continuous movement, with outward side, frontward and upward displacements.

## Patentansprüche

1. Sturzhelm von der Art, die eine Sturzhelmhaube (1) und einen beweglichen Kinnstützen-Visier-Aufbau (2) einschließlich eines Kipp-Kinnstützenvisiervorderteils aufweist, wobei die Sturzhelmhaube (1) beschränkende, auseinanderspreizende, gleitende und sperrende Elemente für den Aufbau trägt, wobei der Aufbau ferner an seinen beiden Endabschnitten im Wesentlichen kreissektorförmige Elemente (3) umfasst, die fest an der Sturzhelmhaube (1) verankert sind, **dadurch gekennzeichnet, dass** der bewegliche Kinnstützen-Visier-Aufbau (2) in einer bündigen Beziehung mit der Sturzhelmhaube (1) angeordnet ist, dass die beschränkenden, auseinanderspreizenden, gleitenden und sperrenden Elemente entlang im Wesentlichen Kreisbahnmustern beweglich sind, welche ideale Drehzentren (2a, 2ab, 2ac) aufweisen, die in einem Bereich angeordnet sind, welcher dazu geeignet ist, ein Ohr eines Benutzers abzudecken, und dass die kreissektorförmigen Elemente (3) gestatten, dass die Benutzerohrbereiche des Sturzhelms von einem Sperrmechanismus dafür gelöst werden, wodurch ein vergrößerter Raum für die Ohrmuschel und für eine Anbringung von beweglichen Elementen (4) bereitgestellt wird.

2. Sturzhelm nach Anspruch 1, **dadurch gekennzeichnet, dass** die beweglichen Elemente (4), die den Benutzerohrbereich bedecken, vom Dreh- und Führungssystem der beweglichen Elemente des Kinnstützen-Visiers unabhängig sind.

3. Sturzhelm nach Anspruch 1, **dadurch gekennzeichnet, dass** die beweglichen Elemente (4), die die Ohren bedecken, durch sich drehende Öffnungsmittel und Sperrmittel zur Bereitstellung eines Sperrens in einer geschlossenen Stellung im Wesentlichen im Inneren des Systems an den Sturzhelm geklemmt sind, wodurch dem Visier-Kinnstützen-Aufbau gestattet wird, ohne störenden Eingriff mit dem Visier-Kinnstützen-Aufbau und im Wesentlichen in einer bündigen Beziehung in Bezug auf die Sturzhelmhaubenkontur gekippt zu werden.

4. Sturzhelm nach Anspruch 1, **dadurch gekennzeichnet, dass** der Sperrmechanismus ein am beweglichen Element (4) montiertes Metallhakenelement (4a) umfasst, das während seiner Schließbewegung mit einem Blattelement (4c), welches an der Sturzhelmhaube (1) angebracht ist, eingreift und es nach hinten treibt, wobei der Sperrmechanismus auch ein bewegliches Gleitstück (4d) umfasst, das eine Vorspann- und Rückholfeder (4e) umfasst, um zu verursachen, dass das Blattelement (4c) nach hinten getrieben wird, wodurch dem Hakenelement (4a) ein Durchgang gestattet wird, und, durch die Feder (4e) gedrängt, in einen Ausschnitt des Hakenelements (4a) eindringt, wodurch eine sperrende Beziehung bereitgestellt wird.

5. Sturzhelm nach den vorhergehenden Ansprüche, **dadurch gekennzeichnet, dass** das Aufwärtsdrängen des Kinnstützenaufbaus, und die entsprechende Drehbewegung nach hinten, gleichzeitig ein Auseinanderspreizen der Seitenendabschnitte des Kinnstützenaufbaus bereitstellt, wobei der Kinnstützenaufbau zusammen mit dem damit einstückigen Visieraufbau aus seiner mit dem Sturzhelm bündigen Vertiefung gelöst wird, wodurch die Durchführung einer Drehbewegung nach hinten gestattet wird.

6. Sturzhelm nach Anspruch 1, **dadurch gekennzeichnet, dass** die Kreissektorelemente (3) an beiden Seiten davon Radelemente (2b) umfassen, welche Radelemente in ihrem geschlossenen Zustand in einer unteren Aussparung (3c) der Kreissektorelemente (3) in Eingriff stehen, und welche, während sie angetrieben werden und die Aussparung dafür verlassen, gleichzeitig die Seitenabschnitte des Kinnstützenaufbaus drehend auseinanderspreizen, wodurch verursacht wird, dass letztere aus der mit der Sturzhelmhaube bündigen Vertiefung dafür gelöst werden, und die Seitenabschnitte selbst an einer oberen Endlagenposition in einem auseinandergespreizten Zustand gehalten werden.

7. Sturzhelm nach Anspruch 1, **dadurch gekennzeichnet, dass** die Kreissektorelemente (3) an ihren beiden Seiten (2f) zwei Radelemente umfassen, wobei diese Radelemente durch Halterippen gehalten werden, die ein im Wesentlichen kreisförmiges Führungsmuster bilden, das zu dem der auseinanderspreizenden Radelemente (2b) koaxial ist, und entlang dem sie gleiten, wodurch verursacht wird, dass der Kinnstützen-Visier-Aufbau um sein ideales Drehzentrum (2ab oder 2ac) aufwärts und nach hinten gedreht wird, um seine Endlagen- oder Grenzposition zu erreichen.

8. Sturzhelm nach Anspruch 1, **dadurch gekennzeichnet, dass** der Kinnstützenaufbau (2) ein erstes Federband (2g) umfasst, das mit einer zweiten runden Drahtfeder (3f) zusammenwirkt, um den Kinnstützen-Visier-Aufbau fest und elastisch in einer angehobenen Stellung davon zu halten.

9. Sturzhelm nach Anspruch 1, **dadurch gekennzeichnet, dass** der Sturzhelm eine Gleitbahn für den Visierhalteaufbau umfasst, wobei die Gleitbahn das gleiche virtuelle Drehzentrum wie jenes des Kinnstützenaufbaus (2ab) aufweist.

10. Sturzhelm nach Anspruch 9, **dadurch gekennzeichnet, dass** das Visierdrehzentrum an einer Position angeordnet ist, die sich von jener des virtuellen Drehzentrums (2ac) des Visier-Kinnstützen-Aufbaus unterscheidet.

11. Sturzhelm nach Anspruch 1, **dadurch gekennzeichnet, dass** das Kreissektorelement (3), das mit dem Kinnstützenaufbau einen einzelnen Körper bildet, so gestaltet ist, dass es dem bündigen Visier gestattet, nach hinten gekippt und gleitend bewegt zu werden.

12. Sturzhelm nach Anspruch 1, **dadurch gekennzeichnet, dass** der Kinnstützen-Visier-Aufbau an entsprechenden Seitenbereichen davon feste und sichere Sperrmittel umfasst, die dazu gestaltet sind, den Aufbau in einer geschlossenen Stellung zu halten, wobei die Sperrmittel im Wesentlichen ein eingreifendes Rastelement (5d) und ein aufnehmendes Rastelement (5b), wobei das eingreifende Rastelement (5d) durch eine Vorspannfeder (5e), die das eingreifende Rastelement in einer vollständig vorspringenden Position hält und stets in diese zurückholt, gedrängt wird, und ein weiteres Rast- oder Sperrelement (5i) und einen Bowdenzug (5f), gesteuert durch einen Steuerhebel (5g), welcher an einem Halteblock am vorderen Abschnitt des Kinnstützenaufbaus angeordnet ist, umfassen.

13. Sturzhelm nach Anspruch 12, **dadurch gekennzeichnet, dass** das eingreifende Rastelement (5d) einen Kopfabschnitt umfasst, der mit einem geneigten Rinnenmuster eingreift, welches ein Metallband (5b) umfasst, das in dem Block (5a) an der Sturzhelmhaube gehalten wird und als beschränkendes aufnehmendes Rastelement wirkt.

14. Sturzhelm nach Anspruch 12, **dadurch gekennzeichnet, dass** der Zug (5f) durch zwei Spannrollen (5h) in einem gespannten Zustand gehalten wird.

15. Sturzhelm nach Anspruch 12, **dadurch gekennzeichnet, dass** das rechte und das linke System der Rastelemente, die vordere Steueranordnung der Rastelemente, einschließlich der Spannelemente und des Schnapperelements des Lufteinlasssystems, alle in drei Blöcken enthalten sind, die durch den Bowdenzug (5f) gekoppelt sind.

16. Sturzhelm nach Anspruch 12, **dadurch gekennzeichnet, dass** der Sturzhelm ein Schnapperelement (6b) umfasst, das das gleiche Drehzentrum (6g) wie jenes des Steuerhebels (5g) aufweist.

17. Sturzhelm nach Anspruch 12, **dadurch gekennzeichnet, dass** ein Abschnitt eines Unter-Kinn-Aufbaus (6d) aus einem biegsamen Material besteht und auch einen Teil bildet, der die Vertiefung enthält, um dem Steuerhebel (5g) den Betrieb zu gestatten, und der, in der abgesenkten Position, eine ausreichende Länge aufweist, um als aerodynamischer Spoiler zu wirken, um Bodenluft an einem Eindringen zu hindern, und der, während er einwärts gedreht ist, in einem Eingriffssitz eingreift, wodurch er in einer bündigen Beziehung mit einer inneren Kontur des Kinnstützenaufbaus gehalten wird.

18. Sturzhelm nach Anspruch 1, **dadurch gekennzeichnet, dass** der Sturzhelm ferner eine Gleitbahn (3 bis) umfasst, die Nockenelemente (3c) umfasst, welche dem Kinnstützenaufbau (2) gestatten, eine zusammengesetzte fortlaufende Bewegung mit Verschiebungen zu einer Außenseite, nach vorne und nach oben bereitzustellen.

## Revendications

1. Casque antichocs d'un type présentant une coiffe de casque antichocs (1) et un ensemble mobile de mentonnière-visière (2) incluant une partie avant de mentonnière-visière pouvant basculer, ladite coiffe de casque antichocs (1) supportant des éléments de retenue, d'écartement, de coulissement et de verrouillage pour ledit ensemble, ledit ensemble comprenant en outre, à ses deux parties d'extrémité, des éléments sensiblement en forme de secteur circulaire (3) solidement ancrés dans ladite coiffe de casque antichocs (1), **caractérisé en ce que** ledit ensemble mobile de mentonnière-visière (2) est agencé en une relation d'affleurement avec ladite coiffe de casque antichocs (1), **en ce que** lesdits éléments de retenue, d'écartement, de coulissement et de verrouillage sont mobiles le long d'empreintes de course sensiblement circulaires, ayant des centres de rotation idéaux (2a, 2ab, 2ac) agencés à l'intérieur d'une région appropriée pour couvrir l'oreille d'un utilisateur, et **en ce que** lesdits éléments en forme de secteur circulaire (3) permettent de dégager la région des oreilles de l'utilisateur dudit élément antichocs d'un mécanisme de verrouillage, en fournissant ainsi un espace agrandi pour les pavillons des oreilles et pour une application d'éléments mobiles (4).

2. Casque antichocs selon la revendication 1, **caractérisé en ce que** lesdits éléments mobiles (4) couvrant la région des oreilles de l'utilisateur sont indépendants du système de mise en rotation et de guidage de l'élément mobile de la mentonnière-visière.

3. Casque antichocs selon la revendication 1, **caractérisé en ce que** lesdits éléments mobiles (4) couvrant lesdites oreilles sont fixés sur la coiffe de casque antichocs grâce à un moyen d'ouverture rotatif et un moyen de verrouillage afin de fournir un verrouillage dans une position de fermeture, sensiblement à l'intérieur du système en permettant à l'ensemble de mentonnière-visière de basculer, sans interférer avec ledit ensemble de mentonnière-visière, et sensiblement avec une relation d'affleurement par rapport au contour de la coiffe de casque antichocs.

4. Casque antichocs selon la revendication 1, **caractérisé en ce que** ledit mécanisme de verrouillage comprend un élément de crochet en métal (4a) assemblé sur ledit élément mobile (4) qui, pendant son mouvement de fermeture, s'engage dans, et entraîne vers l'arrière, un élément de lame (4c) monté sur ladite coiffe de l'élément antichocs (1), ledit mécanisme de verrouillage comprenant également un coulisseau mobile (4d) incluant un ressort de poussée et de rappel (4e), pour que l'élément de lame (4c) soit entraîné vers l'arrière, ce qui permet ainsi au dit élément de crochet (4a) de passer à travers, et, en étant poussé par ledit ressort (4e), de pénétrer dans une découpe dudit élément de crochet (4a) en fournissant une relation de verrouillage.

5. Casque antichocs selon les revendications précédentes, **caractérisé en ce que** la poussée vers le haut dudit ensemble de mentonnière, et le mouvement de rotation vers l'arrière correspondant, fournissent une séparation simultanée des parties d'extrémités latérales dudit ensemble de mentonnière, ledit ensemble de mentonnière étant dégagé de son évidement, en affleurement avec le casque antichocs, en même temps que l'ensemble de visière qui fait partie intégrante de celui-ci, en permettant ainsi d'effectuer un mouvement de rotation vers l'arrière.

6. Casque antichocs selon la revendication 1, **caractérisé en ce que** lesdits éléments de secteur circulaire (3) comprennent des éléments de roulette (2b) sur chacun de leurs côtés, lesquels éléments de roulette, dans un état fermé, s'engagent dans un évidement de fond (3c) desdits éléments de secteur circulaire (3) et lesquels, lorsqu'ils sont entraînés et sortent dudit évidement pour ce faire, séparent simultanément par rotation les parties latérales de l'ensemble de mentonnière en provoquant ainsi le dégagement de celui-ci de l'évidement en affleurement de la coiffe du casque antichocs pour ce faire, et en maintenant lesdites parties latérales dans un état séparé même au niveau d'une extrémité supérieure de la position de course.

7. Casque antichocs selon la revendication 1, **caractérisé en ce que** lesdits éléments de secteur circulaire (3) comprennent deux éléments de roulettes de chaque côté de ceux-ci (2f), lesdits éléments de roulette étant retenus par des nervures de retenue formant une empreinte de guidage sensiblement circulaire, coaxiale à celle des éléments de roulettes de séparation (2b), et le long de laquelle ils coulissent, en provoquant ainsi la rotation dudit ensemble de mentonnière-visière autour de son centre de rotation idéal (2ab ou 2ac) vers le haut et vers l'arrière, afin d'atteindre une fin de course ou une position limite de celle-ci.

8. Casque antichocs selon la revendication 1, **caractérisé en ce que** ledit ensemble de mentonnière (2) comprend un premier ressort en lame (2g) coopérant avec un second ressort en fil rond (3f) afin de maintenir solidement et élastiquement ledit ensemble de mentonnière-visière dans une position relevée de celui-ci.

9. Casque antichocs selon la revendication 1, **caractérisé en ce que** ledit casque antichocs comprend une voie de coulissement pour l'ensemble support de visière, ladite voie de coulissement ayant le même centre de rotation virtuel que celui dudit ensemble de mentonnière (2ab).

10. Casque antichocs selon la revendication 9, **caractérisé en ce que** le centre de rotation de la visière est agencé dans une position différente de celui du centre (2ac) de rotation virtuel de l'ensemble de mentonnière-visière.

11. Casque antichocs selon la revendication 1, **caractérisé en ce que** ledit élément de secteur circulaire (3) formant un seul corps avec ledit ensemble de mentonnière est conçu de façon à permettre à la visière en affleurement de basculer vers l'arrière et de se déplacer par coulissement.

12. Casque antichocs selon la revendication 1, **caractérisé en ce que** ledit ensemble de mentonnière-visière comprend, sur ses régions latérales correspondantes, des moyens de verrouillage solides et sûrs conçus pour retenir ledit ensemble en position fermée, ledit moyen de verrouillage comprenant sensiblement un élément d'enclenchement mâle (5d) et un élément d'enclenchement femelle (5b), ledit élément d'enclenchement mâle (5d) étant poussé par un ressort de poussée (5e) maintenant et recouvrant toujours ledit élément d'enclenchement mâle dans une position totalement en saillie, et un autre élément d'enclenchement ou de verrouillage (5i) et un câble Bowden (5f) commandé par un levier de commande (5g) agencé sur un bloc support au niveau de la partie avant de l'ensemble de mentonnière.

13. Casque antichocs selon la revendication 12, **caractérisé en ce que** ledit élément d'enclenchement mâle (5d) comprend une partie de tête qui s'engage dans un empreinte de goulotte inclinée, comprenant une bande de métal (5b) supportée par ledit bloc (5a) sur la coiffe du casque antichocs, et fonctionnant comme élément d'enclenchement femelle de retenue.

14. Casque antichocs selon la revendication 12, **caractérisé en ce que** ledit câble (5f) est maintenu dans un état de tension par deux galets tendeurs (5h).

15. Casque antichocs selon la revendication 12, **caractérisé en ce que** les systèmes gauche et droite
desdits éléments d'enclenchement, l'agencement de commande avant desdits éléments d'enclenchement, y compris lesdits éléments tendeurs, et l'élément de porte du système d'admission d'air, sont tous inclus dans trois blocs, couplés par ledit câble Bowden (5f).

16. Casque antichocs selon la revendication 12, **caractérisé en ce que** ledit casque antichocs comprend un élément de porte (6b) ayant le même centre de rotation (6g) que celui dudit levier de commande (5g).

17. Casque antichocs selon la revendication 12, **caractérisé en ce qu'**une partie d'un ensemble de jugulaire (6d) est constitué d'un matériau souple, et constitue également une partie incluant l'évidement afin de permettre au dit levier de commande (5g) de fonctionner et qui, dans une position abaissée, a une longueur suffisante pour fonctionner comme un becquet aérodynamique afin d'empêcher que l'air provenant du bas ne pénètre et qui, alors qu'il est tourné vers l'intérieur, s'engage dans un siège d'engagement, en étant ainsi maintenu en une relation d'affleurement avec un contour interne de l'ensemble de mentonnière.

18. Casque antichocs selon la revendication 1, **caractérisé en ce que** ledit casque antichocs comprend en outre une voie de coulissement (3 bis), comprenant des éléments de came (3c) permettant au dit ensemble de mentonnière (2) de fournir un mouvement composite continu, avec des déplacements latéraux vers l'extérieur, vers l'avant et vers le haut.
